(19) Europäisches Patentamt
European Patent Office
Office européen des brevets

(11) Veröffentlichungsnummer : **0 090 978**
**B1**

(12) # EUROPÄISCHE PATENTSCHRIFT

(45) Veröffentlichungstag der Patentschrift : **30.11.88**

(51) Int. Cl.⁴ : **C 07 D403/10**, A 61 K 31/41

(21) Anmeldenummer : **83102669.5**

(22) Anmeldetag : **18.03.83**

(54) **Imidazolylphenyl-tetrahydropyridazine, Verfahren zu ihrer Herstellung und diese enthaltende pharmazeutische Präparate.**

(30) Priorität : **02.04.82 DE 3212304**

(43) Veröffentlichungstag der Anmeldung : **12.10.83 Patentblatt 83/41**

(45) Bekanntmachung des Hinweises auf die Patenterteilung : **30.11.88 Patentblatt 88/48**

(84) Benannte Vertragsstaaten : **AT BE CH DE FR GB IT LI LU NL SE**

(56) Entgegenhaltungen :
**EP-A- 0 075 436**
**DE-A- 2 151 216**

(73) Patentinhaber : **A. Nattermann & Cie. GmbH**
**Nattermannallee 1**
**D-5000 Köln 30 (DE)**

(72) Erfinder : **Hilboll, Gerd, Dr.**
**Dehmelstrasse 36**
**D-5000 Köln 30 (DE)**
Erfinder : **Doppelfeld, Ilse-Stephanie**
**Im Bruchfeldchen 35**
**D-5010 Bergheim-Glessen (DE)**
Erfinder : **Prop, Gerrit, Dr.**
**Mohnblumenweg 25**
**D-5024 Pulheim (DE)**

(74) Vertreter : **Redies, Bernd, Dr. rer. nat.**
**COHAUSZ & FLORACK Patentanwaltsbüro Schumannstrasse 97 Postfach 14 01 47**
**D-4000 Düsseldorf 1 (DE)**

**EP 0 090 978 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Imidazolylphenyl-tetrahydropyridazine und deren physiologisch verträgliche Säureadditionssalze, Verfahren zu ihrer Herstellung, und diese Verbindungen enthaltende pharmazeutische Zubereitungen und ihre Verwendung bei der Prophylaxe und Therapie thrombo-embolischer Erkrankungen, besonders bei hohem Blutdruck.

6-(Acylaminophenyl)-3-oxo-2,3,4,5-tetrahydropyridazine sind bereits mehrfach beschrieben worden. Beispielsweise werden in der DE-OS 2 727 481 diese in der Alkanoylgruppe durch ein oder mehrere Halogenatome substituierten Verbindungen mit thrombozytenaggregationshemmenden und blutdrucksenkenden Eigenschaften beschrieben. Gleiche Eigenschaften werden auch für die Carbamate der DE-OS 3 022 177 genannt.

Aus EP-A 0 075 436 sind substituierte 4,5-Dihydro-6-phenyl-3(2H)-pyridazine und 6-Phenyl-3(2H)-pyridazinone mit kardiotoner Wirksamkeit bekannt.

Es wurde nun gefunden, daß 6-(Imidazolylphenyl)-3-oxo-tetrahydropyridazine der allgemeinen Formel I

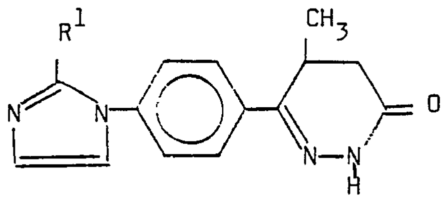

(I)

worin $R^1$ Wasserstoff oder Methyl sind, wertvolle pharmakologische Eigenschaften aufweisen.

Darin enthalten sind auch die Säureadditionssalze von Verbindungen der Formel I. Säureadditionssalze sind insbesondere pharmazeutisch verwendbare, z. B. solche mit Chlorwasserstoffsäure, Bromwasserstoffsäure, Schwefelsäure oder Phosphorsäure, oder mit organischen Säuren, wie entsprechende Carbonsäure, z. B. Essigsäure, Propionsäure, Oxalsäure, Malonsäure, Glykolsäure, Bernsteinsäure, Maleinsäure, Fumarsäure, Äpfelsäure.

Die Verbindungen der Formel I besitzen ein Chiralitätszentrum an der Position 5 des Pyridazinringes und können somit als Racemate oder in Form der Enantiomeren vorliegen. Falls eine Trennung der Racemate erwünscht ist, wird diese zweckmäßigerweise nach an sich bekannten Verfahren mit einer optisch aktiven Säure, wie z. B. Dibenzyolweinsäure oder Campher-10-sulfonsäure, über die Bildung diastereomer Salze oder durch Chromatographie an optisch aktivem Säulenmaterial durchgeführt.

Erfindungsgemäße Verbindungen sind beispielsweise :

6-[4-(1-Imidazolyl)-phenyl]-5-methyl-3-oxo-2,3,4,5-tetrahydro-pyridazin

6-[4-(2-Methyl-1-imidazolyl)-phenyl]-5-methyl-3-oxo-2,3,4,5-tetrahydro-pyridazin.

Die erfindungsgemäßen Verbindungen der Formel I zeichnen sich durch thrombozytenaggregationshemmende und insbesondere blutdrucksenkende Eigenschaften aus. Sie sind als Antihypertensiva und zur Prophylaxe und Therapie thrombo-embolischer Erkrankungen geeignet.

Die Darstellung der erfindungsgemäßen Substanzen der Formel I erfolgt durch Umsetzung von 4-[4-(1-Imidazolyl)-phenyl]-4-oxo-buttersäuren der Formel II in der $R^1$ die in Formel I angegebene Bedeutung haben oder deren Alkylestern, mit einer Hydrazinverbindung der Formel III, deren Hydrat oder Salze, wie Hydrochlorid, Hydrogensulfat, Sulfat u. a., in wässrigen, wässrig-alkoholischen Medien oder in unter den gewählten Bedingungen indifferenten organischen Lösungsmitteln, wie z. B. Dioxan, Toluol, Dimethylformamid, bei Temperaturen von 60 bis 150 °C, vorzugsweise bei 80 bis 100 °C, in Ethanol oder Wasser. Die Umsetzung wird durch folgende

Reaktionsgleichung veranschaulicht :

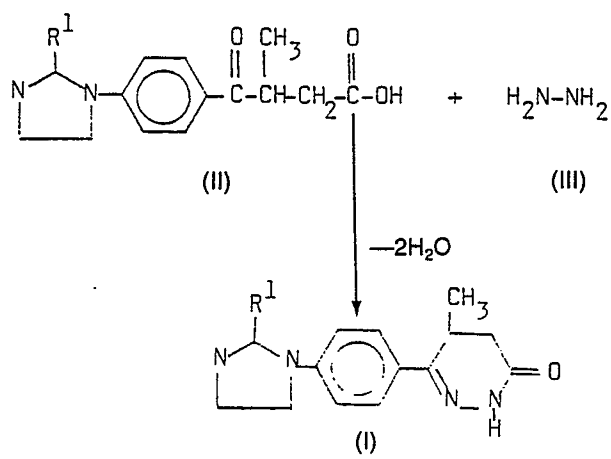


(II) (III)

(I)

Als Ausgangsverbindungen der Formel II kommen infrage :
4-[4-(1-Imidazolyl)-phenyl]-3-methyl-4-oxo-buttersäure,
4-[4-(2-Methyl-1-imidazolyl)-phenyl]-3-methyl-4-oxo-buttersäure.

Die Darstellung der Ausgangsverbindungen der Formel II erfolgt nach an sich bekannten Verfahren :
4-(1-Imidazolyl)-benzaldehyde der Formel IV (s. L. M. Sitkina, A. M. Simonov, C. A. 65 (1966) 13 686e) werden unter dem katalytischen Einfluß von Natriumcyanid (H. Stetter, Angew. Chem. 88 (1976), 695-735) mit den 2-Alkensäurenitrilen der Formel V zu den

4-[4-(1-Imidazolyl)-phenyl]-4-oxo buttersäurennitrilen der Formel VI umgesetzt, die durch Hydrolyse mit Salzsäure in die 4-[4-(1-Imidazolyl)-phenyl]-4-oxo-buttersäuren der Formel II umgewandelt werden. Die Umsetzung wird durch folgendes Formelschema verdeutlicht :

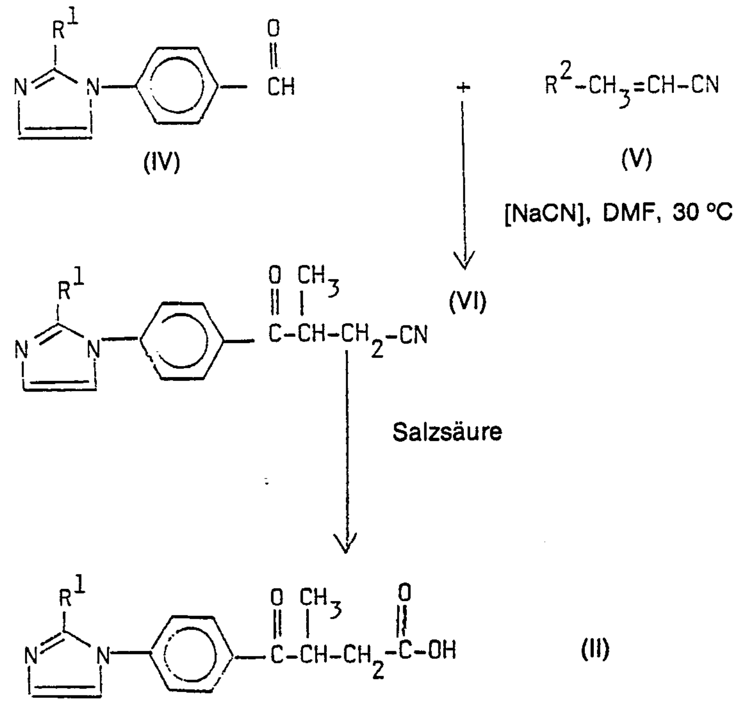


(IV) (V) (VI) (II)

Die Säureadditionssalze von Verbindungen der Formel I mit anorganischen oder organischen Säuren lassen sich durch Mischen der zugrundeliegenden Imidazolylverbindungen mit den entsprechenden Säuren in wässrigen, wässrig-organischen (z. B. Alkohol-Wasser) oder organischen Medien, wie z. B. Alkoholen, Alkohol-Ether-Mischungen oder Ether-Petrolether-Mischungen bei Temperaturen zwischen 0° und 100 °C herstellen.

Zur Untersuchung der pharmakodynamischen Eigenschaften werden folgende Methoden verwendet :

1. Blutdrucksenkende Wirkung an der narkotisierten Ratte. Zur Testung der blutdrucksenkenden Wirkung wird an Gruppen von je 5 männlichen, wachen, normotensiven Wistar-Ratten die erfindungsgemäße Substanz 6-[4-(1-Imidazolyl)-phenyl]-5-methyl-3-oxo-2,3,4,5-tetrahydro-pyridazin = A im Vergleich mit der Referenzsubstanz 1-Hydrazinophthalazin (Hydralazin) = C oral verabreicht.

Neunzig Minuten nach Applikation werden die Tiere mit 75 mg/kg i. p. Pentobarbital narkotisiert und die Trachea und die Arteria carotis kanüliert. Die gemessenen Blutdruck- und Herzfrequenzwerte sind in der Tabelle 1 zusammengefaßt.

Tabelle 1

| Substanz | Dosierung mg/kg p.o. | n[1)] | mittlerer arterieller Druck mmHg | Herzfrequenz Schläge/min. |
|---|---|---|---|---|
| Kontrolle | - | 19 | 112,8±4,8 | 307±11 |
| A | 3,16 | 5 | 54,8±8,9 | 415±17 |
| C | 3,16 | 5 | 82,0±6 | 356±8 |

[1)] n = Anzahl der Tiere

2. Blutdrucksenkende Wirkung am anaesthesierten Hund. Die Beeinflussung des systolischen und diastolischen Blutdrucks durch die erfindungsgemäßen Substanzen 6-[4-(1-Imidazolyl)-phenyl]-5-methyl-3-oxo-2,3,4,5-tetrahydropyridazin = A und 6-[4-(2-Methyl-1-imidazolyl)-phenyl]-5-methyl-3-oxo-2,3,4,5-tetrahydro-pyridazin = B bei intraduodenaler Verabreichung wurde am anaesthesierten Hund untersucht.

Tabelle 2

| Substanz | Dosierung mg/kg i.d. | Druck △ mmHg systolisch | diastolisch |
|---|---|---|---|
| A | 0,316 | -51,77 | -51,42 |
| B | 0,316 | -48,18 | -43,80 |

3. Hemmung der Collagen-induzierten Thrombozytenaggregation in vitro.

Die Hemmung der Collagen-induzierten Thrombozytenaggregation in vitro wurde nach der Methode von Born (Nature, 194, 927-929 (1962)) in modifizierter Form (Simultanapplikation) am thrombozytrenreichen Humanplasma mit der erfindungsgemäßen Substanz 6-[4-(1-Imidazolyl)-phenyl]-5-methyl-3-oxo-2,3,4,5-tetrahydro-pyridazin = A im Vergleich mit der Referenzsubstanz Acetylsalicylsäure = D durchgeführt.

Als $IC_{50}$ wird die Konzentration bestimmt, welche eine 50 %ige Hemmung der Aggregation verursachte (Tabelle 3).

Tabelle 3

| Substanz | $IC_{50}$ (Mol/l) |
|---|---|
| A | $2,4 \times 10^{-6}$ |
| D | $> 3 \times 10^{-4}$ |

4. Verminderung der Thrombenbildung an narkotisierten Ratten. Die Versuche zur Verminderung der Thrombenbildung durch die erfindungsgemäßen Substanzen 6-[4-(1-Imidazolyl)-phenyl]-5-methyl-3-oxo-2,3,4,5-tetrahydro-pyridazin = A und 6-[4-(2-Methyl-1-imidazolyl)-phenyl]-5-methyl-3-oxo-2,3,4,5-tetrahydro-pyridazin im Vergleich mit der Referenzsubstanz Acetylsalicylsäure = D bei oraler Verabreichung wurden an narkotisierten Ratten durchgeführt.

Tabelle 4

| Substanz | Dosierung mg/kg p.o. | Verminderung in % |
|---|---|---|
| A | 10 | - 52 |
| B | 10 | -48 |
| D | 100 | - 45 |

Die Ergebnisse aus Tabelle 1 zeigen eine starke blutdrucksenkende Wirkung der erfindungsgemäßen Substanz A im Vergleich mit dem bekannten Antihypertonicum Hydralazin = C an der Ratte, während in Tabelle 2 die starke Beeinflussung des Blutdrucks durch die erfindungsgemäßen Substanzen A und B gezeigt wird.

Neben der blutdrucksenkenden Wirkung zeigen die erfindungsgemäßen Substanzen eine starke Hemmung der durch Collagen induzierten Aggregation von Thrombozyten des Menschen in vitro bzw. eine Verminderung der Thrombusbildung an narkotisierten Ratten im Vergleich zu dem bekannten aggregationshemmenden Pharmakon Acetylsalicylsäure = D, wie aus den Tabellen 3 und 4 zu entnehmen ist.

Die vorliegende Erfindung betrifft ebenfalls pharmazeutische Präparate, welche Verbindungen der Formel I oder pharmazeutisch verwendbare Säureadditionssalze dieser Verbindungen enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z. B. Tabletten, Dragees, Kapseln, Suppositorien, Granulate, Lösungen, Emulsionen oder Suspensionen. Die Dosierung der Verbindung liegt üblicherweise zwischen 0,1-500 mg pro Dosis, vorzugsweise zwischen 1-150 mg je Dosis und kann ein- oder mehrmals, bevorzugt zwei- bis dreimal täglich, verabreicht werden.

Die Herstellung der erfindungsgemäßen Verbindungen wird durch die folgenden Beispiele näher erläutert. Die angegebenen Schmelzpunkte wurden mit einem Büchi 510-Schmelzpunktbestimmungsapparat gemessen und sind mit °C angegeben und nicht korrigiert. Die IR-Spektren wurden mit dem Gerät Perkin Elmer 257 und die Massenspektren mit dem Gerät Varian MAT-311-A (70eV) aufgenommen.

## Beispiel 1

6-[4-(1-Imidazolyl)-phenyl]-5-methyl-3-oxo-2,3,4,5-tetrahydropyridazin.

a) 4-[4-(1-Imidazolyl)-phenyl]-3-methyl-4-oxo-buttersäurenitril.

Eine Lösung von 16 g 4-(1-Imidazolyl)-benzaldehyd in 100 ml Dimethylformamid wird in Stickstoffatmosphäre zu einer Mischung aus 0,36 g Natriumcyanid und 40 ml Dimethylformamid zugetropft. Anschließend wird eine Mischung aus 7,5 g Butensäurenitril und 60 ml Dimethylformamid zugegeben und die Mischung 2 Stunden bei 30 °C gerührt. Nach Zugabe von Wasserwird mit Chloroform extrahiert, die organische Phase mit Wasser gewaschen, getrocknet, eingeengt und der Rückstand durch Säulenchromatographie (Kieselgel/Chloroform) gereinigt.

Ausbeute : 7,1 g, Fp. 107 °C

IR (in KBr) : 2252, 1676 $cm^{-1}$

MS [m/e] : 239 ($M^+$, 60 %), 212 (6 %), 171 (100 %), 143 (68 %), 116 (72 %)

b) 4-[4-(1-Imidazolyl)-phenyl]-3-methyl-4-oxo-buttersäure.

7,1 g Oxobuttersäurenitril 1a) werden in 100 ml 18%-iger Salzsäure 3 Stunden unter Rückfluß erhitzt. Anschließend werden Aktivkohle und 100 ml Wasser zugegeben, kurz aufgekocht und heiß filtriert. Nach Abkühlen wird auf pH 6 eingestellt. Die erhaltene Lösung (260 ml) wird ohne Reinigung weiter umgesetzt bis auf 10 ml, die abgetrennt und bis zur Trockne eingeengt werden. Nach Trocknen des Rückstandes wird die Säure durch Dünnschichtchromatographie (Kieselgel//Chloroform/Methanol/Wasser 70/26/4) gereinigt. Fp. 186 °C.

IR (in KBr) : 1705, 1677 $cm^{-1}$

MS [m/e] : 258 ($M^+$, 16 %), 240 (5 %), 171 (100 %), 143 (17 %), 116 (17 %)

c) 6-[4-(1-Imidazolyl)-phenyl]-5-methyl-3-oxo-2,3,4,5-tetrahydro-pyridazin.

225 ml der wässrigen Lösung 1b) werden mit 6,5 ml Hydrazinhydrat 6 Stunden bei 90 °C gerührt. Nach Abkühlen wird mit Chloroform extrahiert, die Chloroformlösung mit Wasser gewaschen, getrocknet und eingeengt. Der Rückstand wird durch Säulenchromatographie (Kieselgel//Chloroform/Methanol) gereinigt und anschließend aus Ethanol umkristallisiert.

Ausbeute : 2,2 g, Fp. 199-200 °C

IR (in KBr) : 1692, 1617 $cm^{-1}$

MS [m/e] : 254 ($M^+$, 100 %), 239 (13 %), 225 (1 %), 211 (4 %), 197 (12 %), 183 (6 %), 169 (11 %), 142 (8 %), 128 (3 %), 115 (13 %), 102 (5 %).

## Beispiel 2

6-[4-(2-Methyl-1-imidazolyl)-phenyl]-5-methyl-3-oxo-2,3,4,5-tetrahydro-pyridazin.

a) 4-[4-(2-Methyl-1-imidazolyl)-phenyl]-3-methyl-4-oxo-buttersäurenitril.

Analog Beispiel 1a) wird die Reaktion durchgeführt mit 18,6 g 4-(2-Methyl-1-imidazolyl)-benzaldehyd (aus 2-Methylimidazol und 4-Brombenzaldehyd, Fp. 108-110 °C), 5,4 g 2-Butensäurenitril und 0,4 g Natriumcyanid in 100 ml Dimethylformamid.

Ausbeute : 20,2 g. Fp. 97-99 °C

IR (in KBr) : 2245, 1680 $cm^{-1}$

MS [m/e] : 253 ($M^+$, 42 %), 185 (100 %), 157 (29 %), 130 (5 %), 116 (21 %)

b) 4-[4-(2-Methyl-1-imidazolyl)-phenyl]-3-methyl-4-oxo-buttersäure.

Analog Beispiel 1b wird die Hydrolyse durchgeführt mit 20 g Oxobuttersäurenitril 2a) in 100 ml 18 %-iger Salzsäure.

Ausbeute : 9,3 g, Fp. 168-170 °C

IR (in KBr) : 1705, 1680 $cm^{-1}$

MS [m/e] : 272 ($M^+$, 23 %), 254 (27 %), 226 (7 %), 185 (100 %), 157 (28 %), 116 (19 %)

c) 6-[4-(2-Methyl-1-imidazolyl)-phenyl]-5-methyl-3-oxo-2,3,4,5-tetrahydro-pyridazin.

Analog Beispiel 1c) wird die Umsetzung durchgeführt mit 7 g Oxobuttersäure 2b) und 1,5 ml Hydrazinhydrat in 100 ml Wasser.

Ausbeute : 5,3 g, Fp. 197-199 °C

IR (in KBr) : 1686, 1612 $cm^{-1}$

MS [m/e] : 268 ($M^+$, 100 %), 253 (9 %), 241 (13 %), 183 (14 %), 115 (18 %).

Beispiel 3

6-[4-(1-Imidazolyl)-phenyl]-5-methyl-3-oxo-2,3,4,5-tetrahydro-pyridazin-Fumarsäuresalz.

Eine Mischung aus 300 mg Pyridazin 1c) und 137 mg Fumarsäure wird in 50 ml Ethanol bis zur Lösung erhitzt. Anschließend wird eingeengt und der Rückstand getrocknet.

Ausbeute : 340 mg, Fp. 214-216 °C (Zers.)

IR (in KBr) : 1704, 1646, 1610 $cm^{-1}$.

Analog Beispiel 3 lassen sich z. B. Oxalate, Succinate, Malonate usw. sowie anorganische Salze, wie Hydrochloride, Hydrogensulfate usw. herstellen.

**Patentansprüche** (für die Vertragsstaaten : BE, CH, DE, FR, GB, IT, LI, LU, NL, SE)

1. Imidazolylphenyl-tetrahydropyridazine der allgemeinen Formel I

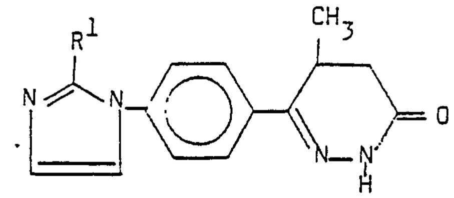

(I)

worin $R^1$ Wasserstoff oder Methyl ist, sowie deren Säureadditionssalze mit anorganischen oder organischen Säuren.

2. 6-[-4-(1-Imidazolyl)-phenyl]-5-methyl-3-oxo-2,3,4,5-tetrahydro-pyridazin und dessen pharmazeutisch verträgliche Salze.

3. 6-[4-(2-Methyl-1-imidazolyl)-phenyl]-5-methyl-3-oxo-2,3,4,5-tetrahydro-pyridazin und dessen pharmazeutisch verträgliche Salze.

4. Verfahren zur Herstellung von Verbindungen gemäß den Ansprüchen 1 bis 3, dadurch gekennzeichnet, daß man eine 4-[4-(1-Imidazolyl)-phenyl]-4-oxo-buttersäure der Formel II

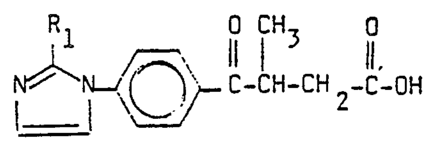

(II)

wobei $R^1$ die in Formel I angegebene Bedeutung hat oder deren Alkylester mit Hydrazin der Formel III

$$H_2N—NH_2 \qquad (III)$$

dessen Hydrat oder ein Salz hiervon, in wässrigen, wässri-alkoholischen oder alkoholischen Medien oder in unter den gewählten Bedingungen indifferenten Lösungsmitteln bei Temperaturen im Bereich von 60° bis 150 °C umsetzt.

5. Verfahren gemäß Anspruch 4, dadurch gekennzeichnet, daß die Reaktion in Dioxan oder Dimethylformamid durchgeführt wird.

6. Verfahren gemäß den Ansprüchen 4 oder 5, dadurch gekennzeichnet, daß die Reaktion bei einer Temperatur im Bereich von 80 bis 100 °C durchgeführt wird.

7. Pharmazeutische Präparate, dadurch gekennzeichnet, daß sie eine Verbindung der Formel I oder deren pharmazeutisch verträgliches Salz gemäß den Ansprüchen 1 bis 3 als Wirkstoff im Gemisch mit üblichen pharmazeutischen Hilfs- und Trägerstoffen enthalten.

**Patentansprüche** (für den Vertragsstaat AT)

1. Verfahren zur Herstellung von Imidazolylphenyltetrahydropyridazinen der allgemeinen Formel I

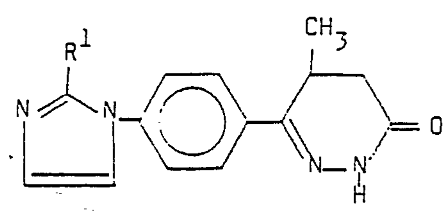

(I)

worin $R^1$ Wasserstoff oder Methyl ist, sowie deren Säureadditionssalze mit anorganischen oder organischen Säuren, dadurch gekennzeichnet, daß man eine 4-[4-(1-Imidazolyl)-phenyl]-4-oxo-buttersäure der Formel II

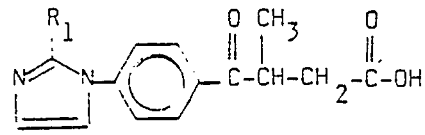

(II)

wobei $R^1$ die in Formel I angegebene Bedeutung hat oder deren Alkylester mit Hydrazin der Formel III

$$H_2N—NH_2 \quad (III)$$

dessen Hydrat oder ein Salz hiervon, in wässrigen, wässrig-alkoholischen oder alkoholischen Medien oder in unter den gewählten Bedingungen indifferenten Lösungsmitteln bei Temperaturen im Bereich von 60° bis 150 °C umsetzt.

2. Verfahren gemäß Anspruch 1, dadurch gekennzeichnet, daß die Reaktion in Dioxan oder Dimethylformamid durchgeführt wird.

3. Verfahren gemäß den Ansprüchen 1 oder 2, dadurch gekennzeichnet, daß die Reaktion bei einer Temperatur im Bereich von 80 bis 100 °C durchgeführt wird.

**Claims** (for the Contracting States : BE, CH, DE, FR, GB, IT, LI, LU, NL, SE)

1. Imidazolylphenyl-tetrahydropyridazines of the general formula I

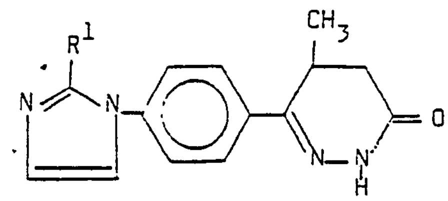

(I)

wherein $R^1$ is hydrogen or methyl, as well as the acid salts thereof with inorganic or organic acids.

2. 6-[-4-(1-Imidazolyl)-phenyl]-5-methyl-3-oxo-2,3,4,5-tetrahydro-pyridazine and the pharmaceutically acceptable salts thereof.

3. 6-[4-(2-Methyl-1-imidazolyl)-phenyl]-5-methyl-3-oxo-2,3,4,5-tetrahydro-pyridazine and the pharmaceutically acceptable salts thereof.

4. Process for the production of the compounds according to claims 1 to 3, characterized in that 4-[4-(1-imidazolyl)-phenyl]-4-oxo-butyric acid of formula II

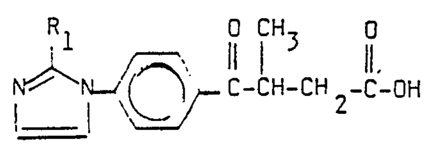

(II)

in which $R^1$ has the meaning given in formula I, or alkyl esters thereof, is reacted with hydrazine of formula III

$$H_2N—NH_2 \quad (III)$$

the hydrate or a salt thereof in aqueous, aqueous-alcoholic or alcoholic media or in solvents which are inert in the chosen conditions at temperatures ranging from 60 to 150 °C.

5. Process according to claim 4, characterized in that the reaction is carried out in dioxane or dimethylformamide.

6. Process according to claims 4 or 5, characterized in that the reaction is carried out at a temperature ranging from 80 to 100 °C.

7. Pharmaceutical products, characterized in that they comprise a compound of formula I or a pharmaceutically acceptable salt thereof according to any of claims 1 to 3 as active agent admixed with usual pharmaceutical auxiliary agents and carriers.

**Claims** (for the Contracting State AT)

1. Process for the production of imidazolylphenyltetrahydropyridazines of the general formula I

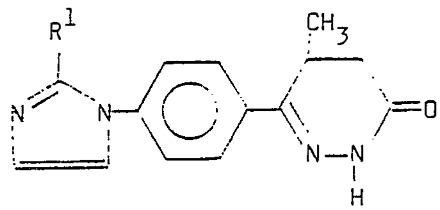

(I)

wherein $R^1$ is hydrogen or methyl, as well as the acid salts thereof with inorganic or organic acids, characterized in that 4-[4-(1-imidazolyl)-phenyl]-4-oxo-butyric acid of formula II

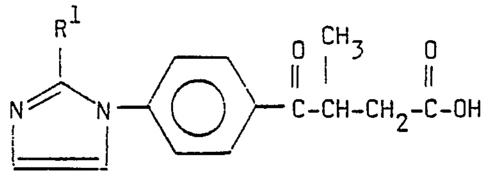

(II)

in which $R^1$ has the meaning given in formula I, or alkyl esters thereof, is reacted with hydrazine of formula III

$$H_2N{-}NH_2 \quad (III)$$

the hydrate or a salt thereof in aqueous, aqueous-alcoholic or alcoholic media or in solvents which are inert in the chosen conditions at temperatures ranging from 60 to 150 °C.

2. Process according to claim 1, characterized in that the reaction is carried out in dioxane or dimethylformamide.

3. Process according to claims 1 or 2, characterized in that the reaction is carried out at a temperature ranging from 80 to 100 °C.

**Revendications** (pour les Etats contractants : BE, CH, DE, FR, GB, IT, LI, LU, NL, SE)

1. Imidazolylphényl-tétrahydropyridazines de formule générale I

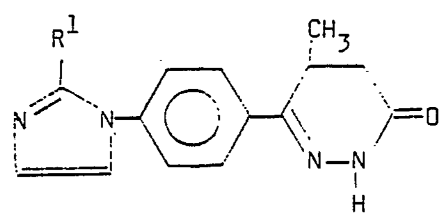

(I)

où R1 est un hydrogène ou un méthyle, ainsi que leurs sels d'addition d'acides avec des acides inorganiques ou organiques.

2. 6-[-4-(1-imidazolyl)-phényl]-5-méthyl-3-oxo-2,3,4,5-tétrahydro-pyridazine et ses sels pharmaceutiquement acceptables.

3. 6-[4-(2-méthyl-1-imidazolyl)-phényl]-5-méthyl-3-oxo-2,3,4,5-tétrahydro-pyridazine et ses sels pharmaceutiquement acceptables.

4. Procédé de préparation de composés selon les revendications 1 à 3, caractérisé en ce qu'on fait réagir un acide 4-[4-(1-imidazolyl)-phényl]-4-oxo-butyrique de formule II

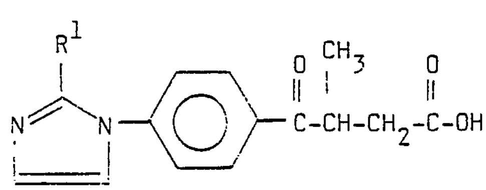

(II)

où R1 a la signification donnée dans la formule I ou son alcoylester avec de l'hydrazine de formule III

$$H_2N{-}NH_2 \quad (III)$$

son hydrate ou un de ses sels, dans des milieux aqueux, alcooliques aqueux ou alcooliques, ou dans des solvants indifférents dans les conditions choisies, à des températures situées dans un intervalle allant de 60° à 150 °C.

5. Procédé selon la revendication 4, caractérisé en ce qu'on conduit la réaction dans le dioxanne ou le diméthylformamide.

6. Procédé selon les revendications 4 ou 5, caractérisé en ce qu'on conduit la réaction à une température située dans un intervalle allant de 80 à 100 °C.

7. Préparations pharmaceutiques caractérisées en ce qu'elles contiennent un composé de formule I ou son sel pharmaceutiquement acceptable selon les revendications 1 à 3 comme substance active mélangée à des additifs et supports pharmaceutiquement habituels.

**Revendications** (pour l'Etat contractant AT)

1. Procédé de préparation d'imidazolylphényl-tétrahydropyridazines de formule générale I

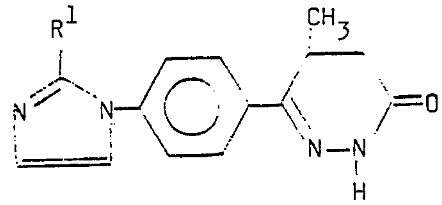

(I)

où R1 représente un hydrogène ou un méthyle, ainsi que de leurs sels d'addition d'acides avec des acides inorganiques ou organiques, caractérisé en ce qu'on fait réagir un acide 4-[4-(1-imidazolyl)-phényl]-4-oxo-butyrique de formule II

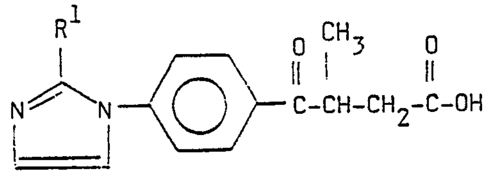

(II)

où R1 a la signification donnée dans la formule I ou son alcoylester avec de l'hydrazine de formule III

$$H_2N—NH_2 \quad (III)$$

son hydrate ou un de ses sels, dans des milieux aqueux, alcooliques aqueux ou alcooliques ou dans des solvants indifférents dans les conditions choisies à des températures situées dans un intervalle allant de 60° à 150 °C.

2. Procédé selon la revendication 1, caractérisé en ce qu'on conduit la réaction dans le dioxanne ou le diméthylformamide.

3. Procédé selon les revendications 1 ou 2, caractérisé en ce qu'on conduit la réaction à une température située dans un intervalle allant de 80 à 100 °C.